# DEMANDE DE BREVET EUROPEEN

(11) **EP 0 938 895 A1**
(43) Date de publication de la demande: **01.09.1999**
(21) Numéro de dépôt: 98430029.3
(22) Date de dépôt: 31.12.1998
(51) Int. Cl.: A61K 9/00, A61K 47/14

(54) **Nouvelle composition destinée à la voie oromuqueuse notamment pernasale**

(30) Priorité: 15.01.1998 FR 9800574
(71) Demandeur: IMMUNOTECH, F-13276 Marseille (FR)
(72) Inventeur: Chauveau, Jacques, 13006 Marseille (FR); Meyer, Pascal, 13003 Marseille (FR); Barbet, Jacques, 13008 Marseille (FR); Delaage, Michel, 13001 Marseille (FR)
(74) Mandataire: Rinuy, Santarelli

(57) **Abrégé**

Une composition pharmaceutique liquide pour administration par voie oromuqueuse, comprenant au moins un principe actif ainsi que des capryl caproyl macrogol glycérides, procédé de préparation et applications.

## Description

La présente invention concerne une nouvelle composition destinée à la voie oromuqueuse notamment pernasale.

La voie d'administration orale de principes actifs médicamenteux notamment solides est de loin la plus répandue. En effet, les formes solides administrées par la voie orale sont particulièrement bien adaptées à un traitement ambulatoire et ce type de formulations offre habituellement une bonne stabilité dans le temps. Toutefois, cette voie d'administration dans laquelle le principe actif est administré par la voie buccale pour être délivré dans l'estomac et l'intestin grêle trouve ses limites, notamment parce qu'un bon nombre de principes actifs sont dégradés dans le tractus gastro-intestinal. De ce fait, il est souvent nécessaire d'administrer au patient une dose de principe actif considérablement plus élevée que la dose réellement nécessaire à l'activité du produit telle qu'elle peut l'être par administration parentérale, intraveineuse, sous-cutanée ou intramusculaire par exemple, ce qui conduit à un accroissement du volume de principe actif à administrer. Il est donc nécessaire de fabriquer considérablement plus de principe actif qu'il n'est réellement nécessaire en théorie pour obtenir l'effet thérapeutique souhaité.

C'est pourquoi on recherche toujours, notamment pour les principes actifs particulièrement dégradés dans le tractus gastro-intestinal, de nouvelles formes pharmaceutiques.

On recherche toujours également des alternatives à l'administration parentérale laquelle met en jeu du matériel stérile ainsi que des conditions d'asepsie lors de l'administration qui en limitent la diffusion commerciale.

Parmi les formulations convenant à une utilisation ambulatoire, on connaît également bien les compositions destinées à une administration par l'intermédiaire de la muqueuse bucco-pharyngée ou nasale.

Les compositions pour la voie nasale sont généralement destinées au traitement des affections des muqueuses nasales et rhinopharyngées, et donc essentiellement réservées à un traitement local. Cette forme pharmaceutique est rarement utilisée pour des utilisations plus générales.

Les compositions pour la voie buccale sont généralement destinées au traitement local des affections des muqueuses buccales comme les aphtes. Cette forme pharmaceutique est également rarement utilisée pour des utilisations systémiques.

Il serait donc souhaitable de disposer d'une formulation pharmaceutique efficace convenant particulièrement à un traitement ambulatoire, convenant notamment aux médicaments qui ont tendance à être particulièrement dégradés par administration orale.

Une telle formulation pharmaceutique ne devrait provoquer ni irritation, ni lésion de la muqueuse.

La pratique de l'industrie pharmaceutique exigeant un grand nombre de contrôles de qualité, il serait aussi souhaitable que la nature d'une telle formulation soit telle qu'elle ne perturbe pas le dosage direct du principe actif, de manière à pouvoir facilement suivre le produit dans une chaîne de fabrication, ou identifier le produit. On éviterait ainsi la phase d'extraction du principe actif, en vue de sa caractérisation.

Il serait aussi souhaitable d'administrer des doses importantes de principes actifs sous des volumes les plus réduits possibles.

Dans la plupart des pathologies, il est souhaitable d'obtenir un effet le plus rapidement possible. C'est pourquoi une formulation pharmaceutique particulièrement avantageuse serait capable de procurer un effet médicamenteux le plus rapide possible, avec en outre une bonne reproductibilité de l'administration.

Or la demanderesse a découvert avec étonnement, que des formulations pharmaceutiques particulières pour la voie oro-muqueuse, notamment pour la voie nasale, sont une réponse particulièrement adaptée aux problèmes évoqués ci-dessus.

C'est pourquoi la présente invention a pour objet une composition pharmaceutique liquide pour administration par voie oro-muqueuse, notamment nasale, caractérisée en ce qu'elle comprend un ou plusieurs principes actifs de nature non polypeptidique, ainsi que moins de 5 % poids/poids de capryl caproyl macrogol glycérides (parfois appelés glycérides polyglycolysés en C₈-C₁₀ saturés).

WO-A-94/08622 décrit des compositions pharmaceutiques comprenant un principe actif et un glycéride polyglycolysé, mais le principe actif est un polypeptide d'environ 32 acides aminés, notamment la calcitonine, et le glycéride polyglycolysé représente en proportion 7% et plus, généralement 35 à 90% en poids et plus de la composition qui est généralement sous forme gélifiée ou solide. Lorsque ces compositions comprennent des capryl caproyl macrogol glycérides comme le Labrasol, ces glycérides représentent 20% et plus de la composition.

Dans la présente invention et dans ce qui suit, par 〈〈 voie oromuqueuse 〉〉, l'on entend la muqueuse tapissant les cavités buccale, nasale ou pharyngée.

Par 〈〈 principe actif de nature polypeptidique〉〉, l'on entend un principe actif comprenant un enchaînement d'au moins 10 acides aminés.

L'administration d'un principe actif par voie oro-muqueuse peut impliquer l'administration d'une dose efficace du principe actif sous forme d'une dose unique ou de doses fractionnées.

Le principe actif peut être de toute nature avec la limitation ci-dessus, et on peut citer par exemple des antibiotiques, des bactériostatiques, des antihistaminiques, des analgésiques, des médicaments destinés à la cardio-angéiologie, tels que des anti-hypertenseurs, des diurétiques, des vasodilatateurs, des vasoconstricteurs. Le principe actif peut être aussi destiné à l'endocrinologie et on peut à cet égard citer des oestrogènes, des oestroprogestatifs, des glucocorticoïdes, des hormones hypothalamiques ou hypophysaires ou des progestatifs. Il peut s'agir également de principes actifs destinés à l'infectiologie tels que des antibiotiques ou des antibactériens, des antiviraux ou des vaccins. Il peut s'agir également de composés destinés à la neurologie, tels que des analgésiques.

Le principe actif peut particulièrement être un composé dérivé d'un médiateur endogène, notamment un de ceux décrits dans EP-A-0 457 701 c'est-à-dire un dérivé de molécules biologiquement actives comportant une fonction amine primaire et un noyau hydroxylé ou un de leurs sels d'addition avec les acides minéraux ou organiques, de formule (I):

[R'R''N-A-B-O-CH₂-CO]ₙR₁ (I)

ou de formule (II)

R'R''N-A-B-O-CH₂-CO-NH-R-NH-CO-CH₂-O-B-A-N R'R'' (II)

dans lesquelles
n représente un nombre entier de 1 à 10 ; A représente une chaîne alcoylène linéaire ou ramifiée renfermant de 1 à 5 atomes de carbone ; B représente un noyau aromatique comportant de 6 à 10 atomes de carbone le cas échéant substitués et éventuellement un hétéroatome ; R₁ représente un reste aminé, un reste alcool choisi parmi les phénols éventuellement substitués et les alcools aliphatiques en C₁-C₁₆, R' et R'' représentent un radical alcoyle renfermant de 1 à 5 atomes de carbone ou un atome d'hydrogène, et R représente un reste divalent de diamine ou de polyamine, en particulier les dérivés cités comme préférés dans ce brevet et tout particulièrement le Tryptamine-5-O-Carboxyméthyl-Tyrosyl-Glycinamide (IS 159), ci-après nommé 〈〈 IS 159 〉〉 ou un de ses sels.

Le principe actif peut aussi particulièrement être un principe actif antimigraineux tel un triptan comme le sumatriptan ou le zolmitriptan.

Le principe actif peut représenter pondéralement d'importantes quantités dans la formulation, jusqu'à la limite de solubilité du principe actif dans ladite formulation.

Les capryl caproyl macrogol glycérides sont également connus sous le nom de glycérides en C₈-C₁₀ polyglycolysés saturés. Certains sont commercialisés sous le nom de Labrasol® par la société GATTEFOSSE.

Les capryl caproyl macrogol glycérides sont des mélanges de monoesters, diesters, et triesters du glycérol et des monoesters et diesters de macrogol avec un poids moléculaire moyen compris entre 200 et 400.

Ces composés peuvent être obtenus par alcoolyse partielle de triglycérides à chaîne de longueur moyenne, en utilisant du macrogol, ou par estérification de glycérol et de macrogol avec l'acide caprylique et l'acide caprique ou d'un mélange d'esters de glycérol et d'un condensé d'oxyde d'éthyléne avec l'acide caprylique et l'acide caprique.

Dans des conditions préférentielles de réalisation de l'invention, on utilise moins de 3 % poids/poids de capryl caproyl macrogol glycérides dans une composition pharmaceutique terminée, de préférence de 0,5 à 2,5 %, et tout particulièrement de 1,5 à 2,2 %.

Dans d'autres conditions préférentielles de mise en oeuvre de l'invention, la composition ci-dessus comprend en outre au moins un agent conservateur. L'agent conservateur utilisable est tout conservateur adapté procurant une composition homogène, et tout particulièrement un benzoate alcalin, notamment le benzoate de sodium. L'utilisation de benzoate de sodium à titre de conservateur donne des résultats particulièrement intéressants. En effet, par exemple par utilisation d'acide benzoïque ou de parahydroxybenzoate de méthyle et de propyle, on observe une instabilité du mélange se caractérisant par exemple par une floculation ou par un déphasage.

L'agent conservateur peut être présent dans les proportions indiquées dans les différentes pharmacopées, et notamment dans des proportions allant de 0,05 % à 1,5 % en poids pour les benzoates exprimées en acide benzoïque, notamment de 0,1 à 0,3 % en poids et notamment environ 0,2 % en poids dans une composition pharmaceutique terminée.

Dans d'autres conditions préférentielles de réalisation de l'invention, la composition ci-dessus renferme en outre un agent d'isotonicité bien connu de l'état de la technique, et notamment du chlorure de sodium.

Dans le cas de l'utilisation de chlorure de sodium, sa proportion est avantageusement d'environ 9 pour mille en poids dans une composition pharmaceutique terminée.

Dans encore d'autres conditions préférentielles de mise en oeuvre de l'invention, la composition ci-dessus est une composition nasale liquide, caractérisée en ce qu'elle présente un pH compris entre 4 et 9, notamment entre 5 et 8.

Dans encore d'autres conditions préférentielles de mise en oeuvre de l'invention, la composition nasale liquide ci-dessus renferme de 5 à 100 mg/ml d'un principe actif ci-dessus. Une dose unitaire sera par exemple de 0,1 à 20 mg d'un principe actif.

Le principe actif est avantageusement en solution dans la composition.

Le volume final d'une composition liquide selon l'invention est avantageusement ajusté à l'aide d'eau pour préparations injectables.

On peut aussi incorporer aux compositions selon l'invention d'autres excipients habituellement employés dans ces compositions pharmaceutiques de ce type, tels que les véhicules aqueux, les émulsifiants et les véhicules non aqueux.

La présente invention a encore pour objet une composition pharmaceutique liquide ci-dessus caractérisée en ce qu'elle renferme, en solution aqueuse ou en présence de solvant pharmaceutiquement acceptable, une dose efficace par voie pernasale de Tryptamine-5-O-Carboxyméthyl-Tyrosyl-Glycinamide (IS 159) ou de l'un de ses sels et en ce qu'elle présente un pH de 4 à 8, notamment de 6 à 7.

La présente invention a encore pour objet une composition pharmaceutique liquide ci-dessus caractérisée en ce qu'elle présente une concentration de 5 à 100 mg de Tryptamine-5-O-Carboxyméthyl-Tyrosyl-Glycinamide par ml et en ce qu'une dose unitaire comprenne entre 0,1 et 20 mg de Tryptamine-5-O-Carboxyméthyl-Tyrosyl-Glycinamide.

La présente invention a encore notamment pour objet une composition pharmaceutique liquide ci-dessus, pour administration par voie nasale, comprenant environ 4 g de Tryptamine-5-O-Carboxyméthyl-Tyrosyl-Glycinamide (IS 159), 0,9 g de NaCl, 2 g de Labrasol®, 0,1175 g de benzoate de sodium, eau pour préparation injectable qsp 100 g, pH final compris entre pH 6 et pH7.

La présente invention a encore pour objet un procédé de préparation d'une composition ci-dessus décrite, caractérisé en ce que l'on mélange, selon des méthodes connues en elles mêmes, le ou les principes actifs avec des capryl caproyl macrogol glycérides et avec les autres excipients pharmaceutiquement acceptables désirés.

Les compositions selon l'invention possèdent de remarquables propriétés.

Tout d'abord en ce qui concerne la biodisponibilité du principe actif, et si l'on prend comme voie de référence (100 %) la voie sous-cutanée, lors de tests réalisés, alors que par administration d'une quantité égale de principe actif par voie orale on n'obtient que 1 à 5 % de l'effet obtenu par voie sous cutanée, on obtient par voie sublinguale environ 10 % de l'effet obtenu par voie sous cutanée, et mieux encore par voie nasale on obtient un effet égal à environ 50 % de l'effet obtenu par voie sous cutanée.

En outre, l'effet a été obtenu par voie nasale plus rapidement que par la voie sous cutanée.

De plus, les compositions selon l'invention offrent la possibilité de d'administrer des doses importantes de principe actif sous de petits volumes.

En outre, on observe, par utilisation d'une composition selon l'invention, une excellente reproductibilité de l'effet obtenu entre individus différents.

Par ailleurs, par utilisation d'une composition selon l'invention, on observe que celle-ci, après administration nasale, n'a pas tendance à couler, ce qui procure également un bon confort à l'utilisateur, en lui évitant de s'essuyer, voire de s'irriter.

De plus, il est possible de doser le principe actif directement dans la composition selon l'invention, sans avoir besoin de l'extraire. Ces caractérisation et dosage peuvent être réalisés notamment par chromatographie liquide à hautes performances (HPLC) et par spectrométrie ultraviolet.

Les compositions de l'invention conditionnées peuvent se présenter sous les formes classiques utilisées pour les compositions nasales, c'est à dire notamment nébuliseurs, pulvérisateurs prédosés ou non, flacons compte-gouttes, flacons multidoses ou flacons monodoses.

La présente demande a encore pour objet l'utilisation d'un capryl caproyl macrogol glycéride en proportion de moins de 5 % poids/poids dans une composition pharmaceutique liquide pour administration par voie oro-muqueuse.

La présente demande a encore pour objet une méthode d'administration par voie oro-muqueuse d'au moins un principe actif, caractérisèe en ce que l'on mélange, selon des méthodes connues en elles mêmes, le ou les principes actifs avec des capryl caproyl macrogol glycérides et avec les autres excipients pharmaceutiquement acceptables désirés puis administre la composition pharmaceutique obtenue, de préférence sur la muqueuse nasale.

La présente demande a enfin pour objet
- L'utilisation d'un capryl caproyl macrogol glycéride pour augmenter la solubilité d'un produit;
- L'utilisation d'un capryl caproyl macrogol glycéride pour augmenter l'absorption dans le sang d'un principe actif;
- L'utilisation d'un capryl caproyl macrogol glycéride pour augmenter la vitesse d'absorption dans le sang d'un principe actif;
- L'utilisation d'un capryl caproyl macrogol glycéride pour augmenter la biodisponibilité d'un principe actif;
- L'utilisation d'un capryl caproyl macrogol glycéride dans une formulation liquide pour limiter la perte de principe actif due à l'écoulement de ladite formulation liquide;
- L'utilisation d'un capryl caproyl macrogol glycéride dans une formulation pour assurer une meilleure reproductibilité de l'administration nasale;
- Une méthode de traitement d'un humain souffrant ou étant sujet aux migraines qui comprend l'administration d'une composition ci-dessus dans laquelle le principe actif est un agent antimigraineux, notamment par voie pernasale.

La figure 1 représente le résultat de l'analyse par HPLC de la solution de l'exemple 1 dans les conditions spécifiées dans cet exemple.
La figure 2 représente la concentration d'IS 159 en fonction du temps ainsi que l'écart type correspondant.

Les exemples qui suivent illustrent la présente invention.

### Exemple 1 : Exemples de formulations.

### a) Solution pour administration nasale

On introduit 0,9 g de chlorure de sodium et 0,235 g de benzoate de sodium dans 94,67 g d'eau pour préparations injectables. On met sous agitation jusqu'à dissolution complète. On introduit alors 2 g de Labrasol® sous agitation lente. On ajoute alors 4 g d'IS 159 jusqu'à dissolution complète. On filtre alors la solution sur acétate de cellulose à 0,22 µm.

On a alors réparti le soluté obtenu dans des flacons nébuliseurs.

### b) Solution témoin

A titre de solution témoin, on a aussi réalisé des préparations dans lesquelles le Labrasol® a été remplacé par 2 ml d'eau distillée.

### c) Comprimés sublinguaux

On a préparé une formulation ayant la composition suivante :

| | |
|---|---|
| - IS 159 | 10 mg |
| - Labrasol® | 2 mg |
| - Autres excipients : Lactose, cellulose microcristalline, Amidon de maïs prégélatinisé, Silice colloïdale, stéarate de magnésium. | |

### Exemple 2 : Dosage du principe actif en présence de Labrasol® par spectrophotométrie UV-Visible

La solution de l'exemple 1, est diluée au 1/800 dans de l'eau distillée. Les mesures de l'absorbance à 280 nm et à 350 nm sont identiques à celles obtenues en l'absence de Labrasol®.

L'emploi du Labrasol® ne perturbe donc pas le dosage du principe actif par spectrophotométrie UV-Visible.

### Exemple 3 : Dosage et Analyse du principe actif en présence de Labrasol® par HPLC

La solution de l'exemple 1 est diluée au 1/200 par l'éluant isocratique constitué de 90 volumes de Triéthylamine Acide Phosphorique (TPA) pH 2.5 et 10 volumes de d'Acétonitrile (ACN) et analysée par HPLC (Chromatographie Liquide Haute Performance) à 210 nm.

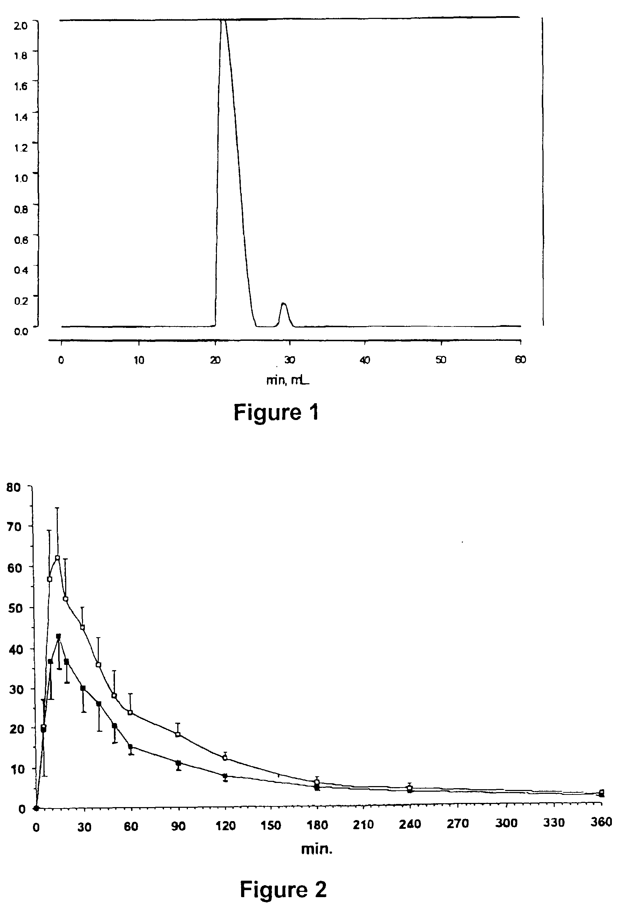

La figure 1 représente le résultat de l'analyse. L'échelle des abcisses représente les paramètres d'élution exprimés en mn (ou ml car le débit est de 1 ml/mn) et l'échelle des ordonnées l'absorbance.

On procède de même avec la solution témoin. Le profil de Chromatographie Liquide Haute Performance et la hauteur des pics sont identiques dans les deux cas.

L'emploi du Labrasol® ne modifie donc pas les critères de l'analyse directe par méthode HPLC du principe actif et du conservateur.

### Exemple 4: Essai de biodisponibilité.

Après administration nasale, chez l'homme d'un volume égal de solution et de solution témoin de l'exemple 1 on a mesuré les concentrations sériques de l'IS-159 en fonction du temps.

La présence du Labrasol® augmente l'absorption (Cmax) de l'IS-159 et diminue le délai d'apparition de l'IS-159 dans le sérum (Tmax apparent observé : témoin 30 min, avec Labrasol® 10 à 15 min.)

L'emploi du Labrasol® augmente considérablement la biodisponibilité de l'IS-159 par voie nasale évaluée par la Surface Sous la Courbe (SSC) de la concentration de l'IS-159 en fonction du temps qui passe, pour une durée d'observation de 4 heures, de 12 à 85 ng/mL/h.

### Exemple 5: Reproductibilité de la biodisponibilité comparée du soluté nasal plus Labrasol®.

On a mesuré les concentrations sériques de l'IS-159 en fonction du temps sur un groupe de 8 sujets après administration nasale d'une quantité identique de 100 µl (soit 4 mg de principe actif IS-159) de solution de l'exemple 1.

Les reproductibilités inter individuelles et intra individuelles des paramètres pharmacocinétiques de IS-159 en soluté nasal en présence de Labrasol® se révèlent bonnes.

On a aussi mesuré les concentrations sériques de l'IS-159 en fonction du temps sur un groupe de 8 sujets après administration nasale de quantités identiques de solution de l'exemple 1 (4 mg de principe actif IS-159) lors de sessions espacées dans le temps.

Les reproductibilités inter-individuelles et intra-individuelles des paramètres pharmacocinétiques de la solution de l'exemple 1 se révèlent également bonnes.

### Exemple 6: Etude de l'effet-dose et reproductibilité de la biodisponibilité comparée.

On a mesuré les concentrations sériques de l'IS-159 en fonction du temps sur un même individu après administration nasale de quantités croissantes de solution de l'exemple 1 (correspondant à 4 mg et 7 mg de principe actif).

On a aussi mesuré les concentrations sériques de l'IS-159 en fonction du temps sur des groupes de 8 sujets après administration nasale de quantités croissantes de solution de l'exemple 1 (correspondant par exemple à 4 mg et 7 mg de principe actif IS-159)

La figure 2 représente la concentration d'IS 159 mesurée exprimée en ng/ml, en fonction du temps exprimé en minutes, ainsi que l'écart type correspondant. Les carrés noirs représentent la dose de 4 mg et les carrés blancs la dose de 7 mg.

Les rapports des SSC et des Concentrations maximales obtenues se révèlent être constants et élevés.

La biodisponibilé et l'absorption de cette formulation nasale se montrent importantes. Cette forte absorption se révèle de plus très rapide.

La bonne reproductibilité intra-individuelle et inter-individuelle des paramètres pharmacocinétiques reste conservée.

### Exemple 7: Comparaison entre formulations sublinguales sans et avec Labrasol®

Le IS-159 a été administré par voie sublinguale.

### a) Dosage et analyse

On a comparé les surnageants de dissolution à l'aide de 1 mL d'eau distillée de comprimés sublinguaux de l'exemple 1 dosés à 10 mg de IS-159 avec 2 % de Labrasol®, et de comprimés sans Labrasol® (2% de lactose).

Après dilution au 1/200 dans de l'eau distillée des surnageants de dissolution, on a mesuré par spectrophotométrie UV-Visible l'absorbance dans les deux cas.

Les mesures de l'absorbance à 280 nm et à 350 nm se sont révélées identiques.

Le Labrasol® ne piège donc pas le principe actif.

L'emploi du Labrasol® ne perturbe pas le dosage de l'IS-159 par spectrophotométrie UV-Visible et n'interfère pas avec le dosage du principe actif contenu dans le comprimé.

Après dilution au 1/50 dans l'éluant isocratique constitué de 90 volumes de Triéthylamine Acide Phosphorique (TPA) pH 2.5 et 10 volumes de d'Acétonitrile (ACN), on a effectué l'analyse HPLC des échantillons.

Le profil HPLC obtenu sur colonne µBondapack est identique dans les deux cas.

L'emploi du Labrasol® ne modifie donc pas les critères de l'analyse directe par méthode HPLC du principe actif.

### b) Biodisponibilité comparée des formulations sublinguales sans et avec Labrasol®.

On a mesuré les concentrations sériques de l'IS-159 en fonction du temps sur des sujets volontaires sains. Ces sujets ont reçu par administration sublinguale une quantité identique de principe actif IS-159 (10 mg) sous forme de comprimés en absence ou en présence de Labrasol® (2 %).

La présence du Labrasol® augmente l'absorption (Cmax) de l'IS-159 et diminue le délai d'apparition de l'IS-159 dans le sérum (Tmax apparent observé : témoin 45 min, avec Labrasol® 35 min.)

L'emploi du Labrasol® augmente considérablement la biodisponibilité de l'IS-159 par voie sublinguale évaluée par la Surface Sous la Courbe (SSC) de la concentration de l'IS-159 mesurée en fonction du temps, pour une durée d'observation de 6 heures, de 0,42 pour le témoin (2% de lactose), à 9,72 ng/mL/h avec Labrasol®.

### Exemple 8: Comparaison entre soluté nasal et formulation sublinguale

On a mesuré les concentrations sériques de IS-159 en fonction du temps sur des sujets volontaires sans. Ces sujets ont reçu une quantité identique de 10 mg d'IS-159 sous forme de comprimés de formule indiquée à l'exemple 7 renfermant 2 % Labrasol® ou par administration nasale de la solution de l'exemple 1.

En présence de Labrasol® et à même concentration de principe actif, le soluté nasal présente une considérablement meilleure biodisponibilité que la formulation sublinguale correspondante. La biodisponibilité de l'IS-159 par voie nasale évaluée par la Surface Sous la Courbe (SSC) des concentrations sériques de l'IS-159 évaluée du temps 0 à l'infini, est de 166 ng/mL/h, contre 12 ng/mL/h pour la formulation sublinguale avec Labrasol® qui elle-même présentait déjà une considérablement meilleure biodisponibilité que la formulation sublinguale sans Labrasol®.

La demi-vie terminale de IS-159 reste constante et voisine de 2 heures et trente minutes.

### Exemple 9: Biodisponibilité comparée soluté nasal/soluté sous cutané.

On a mesuré les concentrations sériques de IS-159 en fonction du temps sur des sujets volontaires sains. Ces sujets ont reçu une quantité identique de 4 mg d'IS-159 par voie sous cutanée (sc) ou par administration nasale de la solution de l'exemple 1.

La biodisponibilité du soluté nasal de IS-159 renfermant du Labrasol® se montre importante et plus rapide que par voie sous cutanée.

La demie vie terminale de IS-159 n'est pas significativement modifiée.

L'administration du soluté nasal se révèle donc reproductible et plus conviviale que la voie d'administration sous-cutanée.

| | SSC/Dose (h.ng/ml)/(mg dose IS-159) | Cmax/dose ng/mL | Tmax en minutes | Demi vie terminale |
|---|---|---|---|---|
| Moyenne nasal | 14 | 11 | 15 | 2,5 h |
| Moyenne s.c. | 36 | 20 | 30 | 2,5 h |
| Nasal / s.c. | ratio = 39 % | ratio = 55% | diminué | non modifié |

### Exemple de comparaison.

On a remplacé dans l'exemple 1, le benzoate de sodium par 0,15 g d'acide benzoïque, 0,2 g de parahydroxybenzoate de méthyle et 0,2 g de parahydroxybenzoate de propyle. Dans les trois cas, on a observé une instabilité de la solution notamment de type floculation ou déphasage.

## Revendications

1. Une composition pharmaceutique liquide pour administration par voie oro-muqueuse, caractérisée en ce qu'elle comprend un ou plusieurs principes actifs de nature non polypeptidique, ainsi que moins de 5 % poids/poids de capryl caproyl macrogol glycérides.

2. Une composition pharmaceutique liquide selon la revendication 1, caractérisée en ce qu'il s'agit d'une formulation pharmaceutique liquide pour administration par voie nasale.

3. Une composition pharmaceutique liquide selon l'une des revendications 1 et 2, caractérisée en ce qu'elle est conditionnée sous une forme utilisée pour les compositions nasales, telle que nébuliseurs, pulvérisateurs prédosés ou non, flacons compte-gouttes, ou flacons monodoses ou multidoses.

4. Une composition pharmaceutique liquide selon l'une des revendications 1 à 3, caractérisée en ce qu'elle renferme moins de 3 % poids/poids de capryl caproyl macrogol glycérides dans une composition terminée.

5. Une composition pharmaceutique liquide selon l'une des revendications 1 à 3, caractérisée en ce qu'elle renferme de 0,5 à 2,5 % poids/poids de capryl caproyl macrogol glycérides dans une composition terminée.

6. Une composition pharmaceutique liquide selon l'une des revendications 1 à 5, caractérisée en ce qu'elle renferme un agent conservateur.

7. Une composition pharmaceutique liquide selon l'une des revendications 1 à 6, caractérisée en ce qu'elle renferme un benzoate alcalin à titre d'agent conservateur.

8. Une composition pharmaceutique liquide selon l'une des revendications 1 à 7 caractérisée en ce qu'elle renferme un dérivé de molécules biologiquement actives comportant une fonction amine primaire et un noyau hydroxylé ou un de leurs sels d'addition avec les acides minéraux ou organiques, de formule (I):
[R'R''N-A-B-O-CH₂-CO]ₙR₁ (I)
ou de formule (II)
R'R''N-A-B-O-CH₂-CO-NH-R-NH-CO-CH₂-O-B-A-N R'R'' (II)
dans lesquelles
n représente un nombre entier de 1 à 10 ; A représente une chaîne alcoylène linéaire ou ramifiée renfermant de 1 à 5 atomes de carbone ; B représente un noyau aromatique comportant de 6 à 10 atomes de carbone le cas échéant substitués et éventuellement un hétéroatome ; R₁ représente un reste aminé, un reste alcool choisi parmi les phénols éventuellement substitués et les alcools aliphatiques en C₁-C₁₆, R' et R'' représentent un radical alcoyle renfermant de 1 à 5 atomes de carbone ou un atome d'hydrogène, et R représente un reste divalent de diamine ou de polyamine.

9. Une composition pharmaceutique liquide selon l'une des revendications 1 à 7 caractérisée en ce qu'elle renferme, en solution aqueuse ou en présence de solvant pharmaceutiquement acceptable, une dose efficace par voie pernasale de Tryptamine-5-O-Carboxyméthyl-Tyrosyl-Glycinamide (IS 159) ou de l'un de ses sels et en ce qu'elle présente un pH de 4 à 8.

10. Une composition pharmaceutique liquide selon la revendication 9 caractérisée en ce qu elle présente un pH de 6 à 7.

11. Une composition pharmaceutique liquide selon la revendication 9 ou 10 caractérisée en ce qu'elle présente une concentration de 5 à 100 mg de Tryptamine-5-O-Carboxyméthyl-Tyrosyl-Glycinamide (IS 159) par ml et en ce qu'une dose unitaire comprenne entre 0,1 et 20 mg de Tryptamine-5-O-Carboxyméthyl-Tyrosyl-Glycinamide (IS 159).

12. Une composition pharmaceutique liquide selon l'une des revendications 1 à 11, pour administration par voie nasale, comprenant environ 4 g de Tryptamine-5-O-Carboxyméthyl-Tyrosyl-Glycinamide (IS 159), 0,9 g de NaCl, 2 g de Labrasol®, 0,1175 g de benzoate de sodium, eau pour préparation injectable qsp 100 g, pH final compris entre pH 6 et pH7.

13. Utilisation d'un capryl caproyl macrogol glycéride en proportion de moins de 5 % poids/poids dans une composition pharmaceutique liquide pour administration par voie nasale comprenant un ou plusieurs principes actifs de nature non polypeptidique.

14. Un procédé de préparation d'une composition pharmaceutique liquide selon l'une des revendications 1 à 12, caractérisé en ce que l'on mélange, selon des méthodes connues en elles mêmes, le ou les principes actifs avec des capryl caproyl macrogol glycérides et avec les autres excipients pharmaceutiquement acceptables désirés.
